Europäisches Patentamt

European Patent Office

Office européen des brevets

(11) Veröffentlichungsnummer: **0 491 145 A2**

# EUROPÄISCHE PATENTANMELDUNG

(21) Anmeldenummer: **91118389.5**

(22) Anmeldetag: **29.10.91**

(51) Int. Cl.5: **A61G 7/047**, A61M 16/00

(30) Priorität: **18.12.90 DE 4040476**

(43) Veröffentlichungstag der Anmeldung:
**24.06.92 Patentblatt 92/26**

(84) Benannte Vertragsstaaten:
**AT BE CH DE DK ES FR GB GR IT LI LU NL SE**

(71) Anmelder: **Hahnen, Werner**
**Krumme Jauchert 1**
**W-7994 Langenargen(DE)**

(72) Erfinder: **Hahnen, Werner**
**Krumme Jauchert 1**
**W-7994 Langenargen(DE)**

(74) Vertreter: **Engelhardt, Guido, Dipl.-Ing.**
**Patentanwalt Montafonstrasse 35 Postfach**
**1350**
**W-7990 Friedrichshafen 1(DE)**

(54) **Einrichtung zur Übertragung von Sauerstoff oder dgl.**

(57) Bei einer Einrichtung zur Übertragung von Sauerstoff und/oder von gasförmigen Medikamenten oder dgl., die aus einer Sauerstoffquelle und/oder einer anderen die Medikamente spendenden Quelle sowie einer Übergabestation besteht, ist zur Schaffung einer Sauerstoffatmosphäre oder einer anderen mit Medikamenten, ätherischen Ölen oder dgl. angereicherten Atmosphäre die Übergabestation als Bettgestell (11), als Matratze (21), als Kissen (31), als Oberbett (41) und/oder als Decke ausgebildet, denen Sauerstoff von der Sauerstoffquelle (1) und/oder die Medikamente von einer entsprechenden Quelle (4) gesteuert und/oder dosiert zuführbar sind. Des weiteren ist die Übergabestation jeweils mit einem oder mehreren Verteilern (12; 22; 32; 42) versehen.

Durch diese Ausgestaltung ist es einem Probanten möglich, die entsprechende Atmosphäre jederzeit, bevorzugt während des Schlafens, zu nutzen, um auf diese Weise eine Behandlung durchzuführen oder Erkrankungen vorzubeugen.

FIG. 1

EP 0 491 145 A2

Die Erfindung bezieht sich auf eine Einrichtung zur Übertragung von Sauerstoff und/oder von gasförmigen Medikamenten oder dgl. und besteht aus einer Sauerstoffquelle und/oder einer anderen die Medikamente spendenden Quelle sowie einer Übergabestation.

Zur Behandlung einer Reihe von Krankheiten, hervorgerufen durch Sauerstoffarmut, beispielsweise von Durchblutungsstörungen, Asthma oder ähnlichen Erkrankungen, ist es bekannt, den Patienten mittels Beatmungsgeräten Sauerstoff zuzuführen. Abgesehen davon, daß der Patient dazu eine in entsprechender Weise ausgerüstete Praxis, meist zu einem vorgegebenen Zeitpunkt, um eine Auslastung der Geräte zu gewährleisten, aufsuchen muß, ist es des weiteren von Nachteil, daß während der Behandlung eine Gesichtsmaske zu tragen ist, mittels der der Sauerstoff dem Patienten zugeführt wird. Derartige Behandlungen sind somit sehr zeitaufwendig und schlecht zu praktizieren, vor allem aber sind diese an einen Ort gebunden und können von einem Patienten nicht nach Bedarf selbst durchgeführt werden, obwohl sie langfristig oder auf Dauer von der Sauerstoffzufuhr abhängig sind. Dadurch geht volkswirtschaftlich Arbeitszeit verloren und die Krankenkassen werden finanziell stark belastet.

Aufgabe der Erfindung ist es daher, eine Einrichtung zur Übertragung von Sauerstoff und/oder von gasförmigen Medikamenten oder dgl. zu schaffen, mittels denen es auf einfache Weise möglich ist, eine entsprechende Atmosphäre zu erzeugen, die durch einen Probanten genutzt werden kann. Die Sauerstoffatmosphäre und/oder eine mit Medikamenten in gasförmigen Zustand angereicherte Atmosphäre soll zu jeder Zeit zur Verfügung stehen, vor allem soll ein Patient beispielsweise eine Sauerstoffbehandlung selbst und zu jeder Zeit und mit geringem finanziellen Mitteln vornehmen können, so daß dafür kein zusätzlicher Zeitaufwand erforderlich ist. Des weiteren soll die Einrichtung einfach in ihrem Aufbau und somit wirtschaftlich herzustellen sein, auch soll diese problemlos bedient werden können.

Gemäß der Erfindung wird dies dadurch erreicht, daß die Übergabestation zur Schaffung einer Sauerstoffatmosphäre oder einer anderen mit Medikamenten, ätherischen Ölen oder dgl. angereicherten Atmosphäre als Bettgestell, als Matratze, als Kissen, als Oberbett und/oder als Decke ausgebildet ist, denen Sauerstoff von der Sauerstoffquelle und/oder die Medikamente von einer entsprechenden Quelle gesteuert und/oder dosiert zuführbar sind, und daß die Übergabestation jeeils mit einem oder mehreren Verteilern versehen ist.

Bei einer als Bettgestell ausgebildeten Übergabestation ist es angebracht, an diesem vorzugsweise in dem oberen Randbereich der Seitenwangen eine oder mehrere Düsenrohre als Verteiler des Sauerstoffes bzw. des Medikamentes vorzusehen und bei Sauerstoffzufuhr das Bettgestell im Bodenbereich mit einer sauerstoffdichten Sperrschicht, beispielsweise in Form einer Holzplatte, auszustatten.

Bei einer als Matratze oder als Kissen oder Decke ausgebildeten Übergabestation sollten in diese eine oder mehrere flexible Zuführungsleitungen vorzugsweise mäanderartig oder kammartig ineinandergreifend als Verteiler angeordnet sein, die mit Austrittsöffnungen versehen oder aus einem sauerstoff- bzw. gasdurchlässigen Werkstoff hergestellt sind.

Bei einer als Oberbett oder als Kissen ausgebildeten Übergabestation ist es zweckmäßig, dieses mit einer Sauerstoff- oder Gaskammer als Verteiler zu versehen, deren Außenseite Austrittsöffnungen aufweist oder aus einem sauerstoff- bzw. gasdurchlässigen Werkstoff besteht.

Die Sauerstoff- oder Gaskammer kann hierbei in einfacher Weise durch eine auf einer sauerstoff- bzw. gasundurchlässig ausgebildeten Beschichtung des Oberbettes oder des Kissens angebrachten Auflage gebildet werden, die in ihren Randbereichen mit dem Oberbett oder dem Kissen verbunden ist.

Vorteilhaft ist es ferner, den Übergabestationen jeweils ein Steuer- oder Dosiergerät vorzuschalten, mittels dem der Sauerstoff und/oder die Medikamente oder dgl. diesen in zeitlichen Intervallen zuführbar ist und den Übergabestationen ionisierter Sauerstoff zuzuführen.

Wird eine Einrichtung zur Übertragung von Sauerstoff und/oder von gasförmigen Medikamenten oder dgl. gemäß der Erfindung ausgebildet, in dem mittels einer in unterschiedlicher Weise gestalteten Übergabestation eine Sauerstoffatmosphäre und/oder eine mit Medikamenten in gasförmigen Zustand angereicherte Atmosphäre geschaffen wird, so ist es einem Probanten möglich, diese Atmosphäre jederzeit, bevorzugt z. B. während des Schlafens, zu nutzen, um auf diese Weise eine Behandlung durchzuführen oder Erkrankungen vorzubeugen. Dazu ist nicht eine entsprechend ausgerüstete Praxis aufzusuchen, vielmehr kann die Sauerstoffbehandlung oder auch eine andere medikamentöse Behandlung in gewohnter Umgebung ohne zeitlichen Aufwand erfolgen. Und da die Atmosphären ohne Schwierigkeiten in zeitlichen Intervallen aufbaubar sind und leicht intensiviert werden können, sind bei einfacher Handhabung, zumal der Sauerstoff in einem erheblichen Maße von der Haut eines Patienten aufgenommen wird, gute Erfolge erzielbar.

In der Zeichnung sind einige Ausführungsbeispiele der gemäß der Erfindung ausgebildeten Einrichtung zur Übertragung von Sauerstoff und/oder von gasförmigen Medikamenten oder dgl. dargestellt, die nachfolgend im einzelnen erläutert sind. Hierbei zeigt:

Figur 1 eine Einrichtung zur Übertragung von Sauerstoff und/oder anderen gasförmigen Medikamtenten mit einer als Bettgestell ausgebildeten Übergabestation,

Figur 2 die Einrichtung nach Figur 1 mit einer als Matratze ausgebildeten Übergabestation,

Figur 3 eine Einrichtung nach Figur 1 mit einer als Kopfkissen ausgebildeten Übergabestation und

Figur 4 eine Einrichtung nach Figur 1 mit einer als Oberbett ausgebildeten Übergabestation.

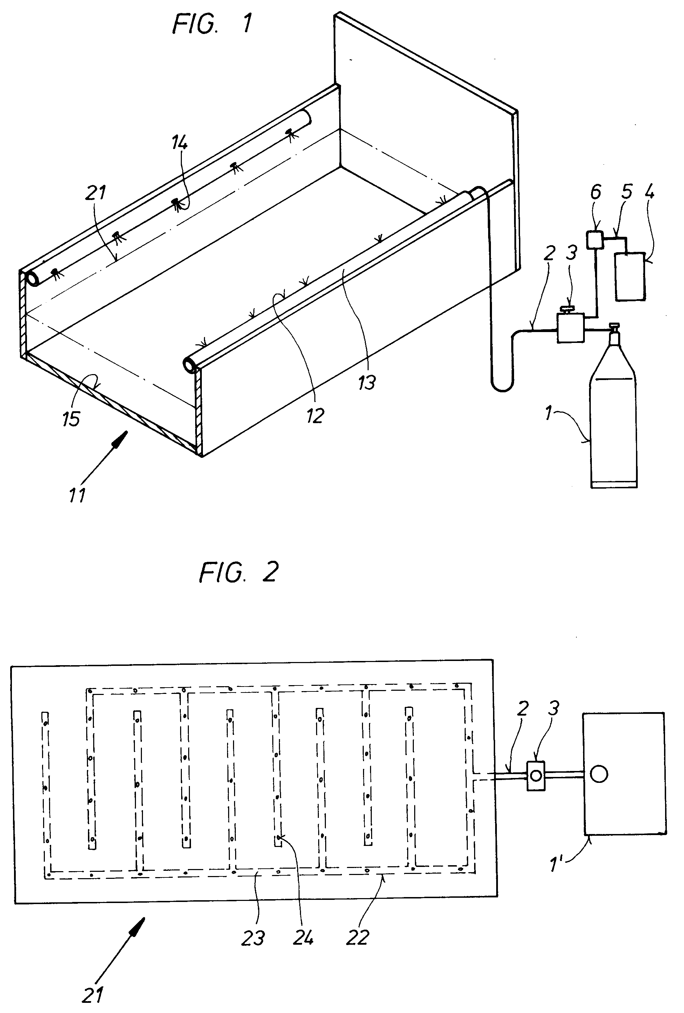

Die in den Figuren 1 bis 4 dargestellte und mit 11, 21, 31 bzw. 41 bezeichneten Einrichtungen dienen jeweils zur Übergabe von Sauerstoff und/oder anderen gasförmigen Medikamenten an einem Probanten und bestehen aus einer Sauerstoffquelle 1 bzw. 1' und einer weiteren Medikamente im gasförmigen Zustand spendenden Quelle 4 sowie unterschiedlich ausgebildeten Übergabestationen, die über eine Leitung 2, in die ein Steuergerät 3 eingebaut ist, mit der Sauerstoffquelle 1 bzw. 1' verbunden sind. Die der Quelle 4 entnehmbaren Medikamente können über eine Leitung 5, die mit einem Dosiergerät 6 versehen ist, ebenfalls dem Steuergerät 3 zugeführt werden. Auf diese Weise kann ohne Schwierigkeiten eine Sauerstoffatmosphäre und/oder eine mit Medikamenten in gasförmigem Zustand angereicherte Atmosphäre geschaffen werden, der sich der Proband mitunter mit seinem gesamten Körper aussetzen kann.

Bei dem Ausführungsbeispiel nach Figur 1 ist die Übergabestation als Bettgestell 11 ausgebildet, das mit einem an die Sauerstoffquelle 1 in Form einer Sauerstoff-Flasche bzw. der Quelle 4 für Medikamente angeschlossenen Verteiler 12, der aus Düsenrohren 13 besteht, versehen ist. Die mit Austrittsöffnungen 14 ausgestatteten Düsenrohre 13 sind hierbei an den Seitenwangen des Bettgestells 11 angebracht, des weiteren ist in dieses im Bodenbereich eine Holzplatte 15 eingelegt oder dieses ist mit einer andersartigen sauerstoffundurchlässigen Sperrschicht versehen. Der aus den Düsenrohren gesteuert, beispielsweise in zeitlichen Intervallen, austretende Sauerstoff sammelt sich somit in dem Bettgestell 11 und steht einem Probanten über einen langen Zeitraum zur Verfügung.

Gemäß Figur 2 ist in der als Matratze 21 ausgebildeten Übergabestation als Sauerstoffverteiler 22 eine flexible Zuführungsleitung 23 angeordnet, die kammartig ineinandergreifende Leitungsteile aufweist und mit Austrittsöffnungen 24 ausgestattet ist. Der einer Sauerstoffquelle 1' in Form eines sauerstofferzeugenden Gerätes entnommene Sauerstoff tritt somit gleichmäßig über die Matratze 21 verteilt aus dieser aus, so daß eine intensive Sauerstoffzuführung über den gesamten Körperbereich eines Probanten möglich ist. Selbstverständlich kann die Matratze 21, wie dies in Figur 1 strichpunktiert eingezeichnet ist, auch in das Bettgestell 11 eingelegt werden.

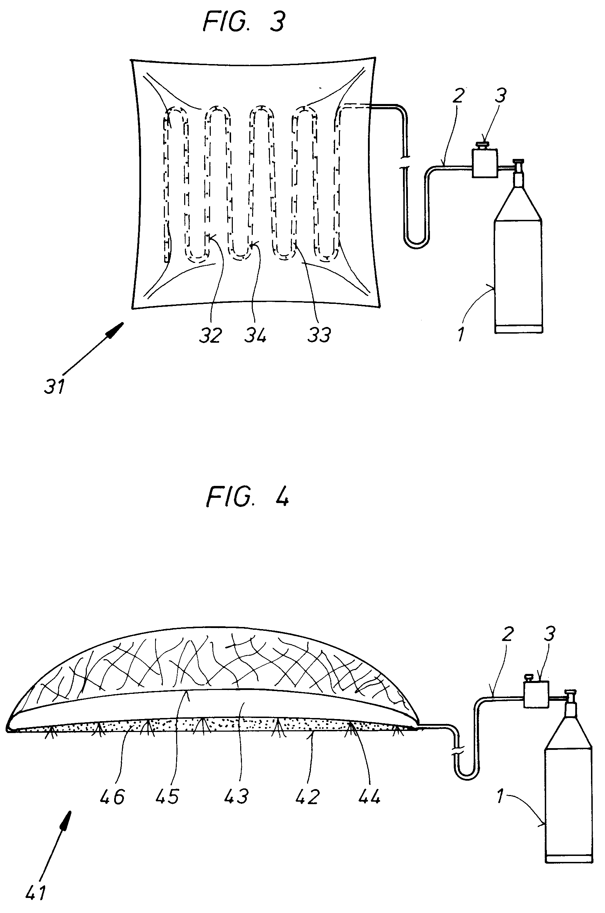

In das in Figur 3 dargestellte Kopfkissen 31 als Übergabestation ist als Verteiler 32 ebenfalls eine flexible mäanderartige Sauerstoffzuführungsleitung 33 eingelegt, die Öffnungen 24 aufweist. Die Sauerstoffzuführungsleitung 23 ist wiederum über die Leitung 2 an eine als Sauerstoff-Flasche ausgebildete Sauerstoffquelle 1 angeschlossen.

Das in Figur 4 dargestellte Oberbett 41 weist einen als Sauerstoffkammer 43 ausgebildeten Verteiler 42 auf. Dazu ist auf einer Seite des Oberbettes 41 eine sauerstoffundurchlässige Beschichtung 45 aufgebracht und auf dieser ist eine Auflage 46 in den Randbereichen befestigt, so daß auf diese Weise die über die Leitung 2 an die Sauerstoffquelle 1 angeschlossene Sauerstoffkammer 43 gebildet ist. Über in der Auflage 46 vorgesehene Öffnungen 44 kann der zugeführte Sauerstoff somit nur auf einer Seite des Oberbettes 41 aus der Sauerstoffkammer 43 ausströmen. Und da die Leitung 2 mit einem Steuergerät 3 ausgestattet ist, kann die Sauerstoffzufuhr auf einfache Weise gesteuert und an die jeweiligen Bedürfnisse angepaßt werden.

**Patentansprüche**

1. Einrichtung zur Übertragung von Sauerstoff und/oder von gasförmigen Medikamenten oder dgl., bestehend aus einer Sauerstoffquelle und/oder einer anderen die Medikamente spendenden Quelle sowie einer Übergabestation,

   **dadurch gekennzeichnet,**

   daß die Übergabestation zur Schaffung einer Sauerstoffatmosphäre oder einer anderen mit Medikamenten, ätherischen Ölen oder dgl. angereicherten Atmosphäre als Bettgestell (11), als Matratze (21), als Kissen (31), als Oberbett (41) und/oder als Decke ausgebildet ist, denen Sauerstoff von der Sauerstoffquelle (1, 1') und/oder die Medikamente von einer entsprechenden Quelle (4) gesteuert und/oder dosiert zuführbar sind, und daß die Übergabestationen jeweils mit einem oder mehreren Verteilern (12; 22; 32; 42) versehen ist.

2. Einrichtung nach Anspruch 1,

**dadurch gekennzeichnet,**

daß bei einer als Bettgestell (11) ausgebildeten Übergabestation an diesem vorzugsweise in dem oberen Randbereich der Seitenwangen (15) eine oder mehrere Düsenrohre (13) als Verteiler (12) des Sauerstoffes oder des Medikamentes vorgesehen sind.

3. Einrichtung nach Anspruch 2,

**dadurch gekennzeichnet,**

daß bei Sauerstoffzufuhr das Bettgestell (11) im Bodenbereich mit einer sauerstoffdichten Sperrschicht, beispielsweise in Form einer Holzplatte (15), versehen ist.

4. Einrichtung nach Anspruch 1,

**dadurch gekennzeichnet,**

daß bei einer als Matratze (21) oder als Kissen (31) oder Decke ausgebildeten Übergabestation in diese eine oder mehrere flexible Zuführungsleitungen (23; 33) vorzugsweise mäanderartig oder kammartig ineinandergreifend als Verteiler (22; 32) angeordnet sind, die mit Austrittsöffnungen (24; 34) versehen oder aus einem sauerstoff- oder gasdurchlässigen Werkstoff hergestellt sind.

5. Einrichtung nach Anspruch 1,

**dadurch gekennzeichnet,**

daß bei einer als Oberbett (41) oder als Kissen (31) ausgebildeten Übergabestation dieses mit einer Sauerstoff- oder Gaskammer (43) als Verteiler (42) versehen ist, deren Außenseite Austrittsöffnungen (44) aufweist oder aus einem sauerstoff- bzw. gasdurchlässigen Werkstoff besteht.

6. Einrichtung nach Anspruch 5,

**dadurch gekennzeichnet,**

daß die Sauerstoff- oder Gaskammer (43) durch eine auf einer sauerstoff- bzw. gasundurchlässig ausgebildeten Beschichtung (45) des Oberbettes (41) oder des Kissens (31) angebrachten Auflage (46) gebildet ist, die in ihren Randbereichen mit dem Oberbett (41) oder dem Kissen (31) verbunden ist.

7. Einrichtung nach einem oder mehreren der Ansprüche 1 bis 6,

**dadurch gekennzeichnet,**

daß den Übergabestationen (11; 21; 31; 41) jeweils ein Steuergerät (3) und/oder ein Dosiergerät vorgeschaltet ist, mittels dem der Sauerstoff und/oder die Medikamente oder dgl. diesen in zeitlichen Intervallen zuführbar ist.

8. Einrichtung nach einem oder mehreren der Ansprüche 1 bis 7,

**dadurch gekennzeichnet,**

daß den Übergabestationen (11; 21; 31; 41) ionisierter Sauerstoff zuführbar ist.

## FIG. 1

## FIG. 2

FIG. 3

31    32    34    33    2    3    1

FIG. 4

41    46    45    43    42    44    2    3    1